# EUROPEAN PATENT SPECIFICATION

(11) **EP 1 126 846 B1**
(45) Date of publication and mention of the grant of the patent: **06.08.2003**
(21) Application number: 99957446.0
(22) Date of filing: 29.10.1999
(51) Int. Cl.: A61K 31/465, A61K 31/40, A61K 31/422, A61K 31/444, A61K 31/325, A61P 25/18, A61P 25/26

(54) **Use of a nicotine receptor agonist for the manufacture of a medicament for the treatment of obsessive compulsive disorder**
Verwendung eines Nikotin-Rezeptor Agonisten zur Herstellung eines Medikaments zur Behandlung von Zwangsneurosen
Utilisation d'un agoniste du recepteur de nicotine pour l'obtention d'un médicament pour le traitement des troubles obsessionnels impulsifs

(30) Priority: 02.11.1998 SE 9803732
(43) Date of publication of application: 29.08.2001
(73) Proprietor: A Carlsson Research AB, 413 19 Göteborg (SE)
(72) Inventor: CARLSSON, Maria, S-413 23 Göteborg (SE); CARLSSON, Arvid, S-413 19 Göteborg (SE)
(74) Representative: Lindberg, Klas Valter Bo
(86) International application number: SE9901948
(87) International publication number: WO00025783

(56) References cited:
- US-A- 5 776 956
- MCCONVILLE B.J. ET AL: 'The Effects of Nicotine Plus Haloperidol Compared to Nicotine Only and Placebo Nicotine Only in Reducing Tic Severity and Frequency in Tourette's Disorder' BIOL PSYCHIATRY vol. 31, 1992, pages 832 - 840, XP002922387
- DAVID J. BALFOUR ET AL: 'Pharmacology of Nicotine and Its Therapeutic Use in Smoking Cessation and Neurodegenerative Disorders' PHARMACOL. THER. vol. 72, no. 1, 1996, pages 51 - 81, XP000900095

## Description

### Field of the invention

The present invention relates to use of nicotine receptor agonists for the manufacture of a medicament for the treatment of obsessive compulsive disorder.

### Background of the invention

Obsessive compulsive disorder (OCD) is a chronic condition. This disorder is normally treated with selective serotonin uptake inhibitors (SSRI) or with the tricyclic antidepressant clomipramine, which is a preferential serotonin uptake inhibitor. The introduction of SSRI has resulted in a marked reduction of anticholinergic side effects and risk of cardiotoxicity compared to the tricyclic compounds. However, SSRI have other side effects that can be bothersome, including sexual and gastrointestinal side effects, nausea, vertigo, headache, palpitations and neurological side effects. Further, some patients experience a superficiality and decreased sharpness in their thinking as well as impaired creativity when taking SSRI. Moreover, although SSRI alleviate many of the symptoms of OCD, they are not a cure. This is not surprising considering the lack of evidence for a primary disturbance in the central serotonergic systems in OCD.

There is thus a need to provide a better cure for OCD, and a drug that lacks the above-described side effects.

In US patent No. 5,776,956 (see column 6) it is suggested that the nicotine metabolite cotinine be used in OCD by virtue of its purported ability to mimic the receptor effects of clomipramine. No clinical data are presented in this patent to support the use of cotinine in OCD. The bulk of evidence from the literature indicates that cotinine does not bind to the nicotine receptor (Yo-shida and Imura, 1979; Abood et al., 1981; Anderson and Arneric, 1994; see Lin and Meyer, 1998), nor mimic the sympathomimetic, biochemical or behavioral effects of nicotine in man (Benowitz et al, 1983) or animals (Mitchell et al., 1989; Goldberg et al., 1989; Toth et al, 1992; Radek, 1993; see Rosecrans, 1979). In a few cases very high doses of cotinine have been shown to have behavioral effects, which were attributed to interaction with non-nicotinic receptors (Goldberg et al., 1989; Keenan et al, 1994; see also Crooks and Dwoskin, 1997, p 747). Goldberg et al. (1989) stress in their paper the difficulties in performing studies with high doses of cotinine, which may contain impurities of nicotine in doses high enough to cause behavioral effects.

Tourette's syndrome (= Gilles de la Tourette's syndrome) is a chronic familial neuropsychiatric disorder characterized by involuntary motor tics and vocalisations. Tourette's syndrome is partially genetically and phenomenologically related to obsessive compulsive disorder. However, OCD and Tourette's syndrome are treated in different ways. In the case of Tourette's syndrome, neuroleptics are used. The symptoms can thus be suppressed by neuroleptics such as haloperidol and pimozide. Neuropsychiatric literature describes an acute reduction of tic frequency and severity in Tourette's syndrome following the use of nicotine chewing gum added to haloperidol; the nicotine chewing gum alone caused an acute reduction in tic frequency (McConville et al, 1992). Also the transdermal nicotine patch has been reported to have beneficial acute effects in Tourette's syndrome (Silver and Sanberg, 1993). Moreover, administration of two 10 mg transdermal nicotine patches during two consecutive days has been found to reduce tics up to four weeks (Dursun et al, 1994).

### Summary of the invention

The object of the present invention is to provide a new, efficient way suitable to treat OCD with a drug lacking the side effects associated with drugs previously used. This is obtained by the use of nicotine or a nicotine receptor agonist for the manufacture of a medicament for the treatment of OCD. According to the invention it is thus possible to reduce both obsessional (e.g. intrusive thoughts) and compulsive (e.g. checking) features of OCD by low daily doses of nicotine.

### Detailed description of the invention

The invention thus relates to the use of a nicotine receptor agonist for the production of a pharmaceutical preparation for the treatment of obsessive compulsive disorder, comprising a nicotine receptor agonist or a pharmaceutically acceptable salt thereof, in an amount effective in alleviating, reducing or abolishing one or several of the symptoms of obsessive compulsive disorder.

The nicotine receptor agonist used according to the invention may be any ligand that binds to and activates the nicotine receptor, thereby resulting in a biological response.

Examples of nicotine receptor agonists according to the invention are nicotine, ethyl nicotine, 3-ethynyl-5-(1-methyl-2-pyrrolidinyl)pyridine (SIB-1765F, SIBIA), 4-[[2-(1-methyl-2-pyrrolidinyl)ethyl]thio]phenol (SIB-1553, SIBIA), (S)- 3-ethynyl-5-(1-methyl-2-pyrrolidinyl)-pyridine (SIB-1508Y, SIBIA), 4'-methylnicotine or (2S-trans)-3-(1,4-dimethyl-2-pyrrolidinyl)pyridine (ABBOT), 2-methyl-3-[(2S)-2-pyrrolidinylmethoxy]pyridine (ABT-089, ABBOT), 3-methyl-5-[(2S)-1-methyl-2-pyrrolidinyl]-isoxazole (ABT-418, ABBOT), 3-PMP or 3-(1-pyrrolidinyl-methyl)pyridine (RJ Reynold), (3E)-N-methyl-4-(3-pyridinyl)-3-buten-1-amine (RJR-2403, RJ Reynold), anabasein or 3,4,5,6-tetrahydro-2,3'-bipyridine (RJ Reynold), 5-fluoronicotine or (S)-5-fluoro-3-(1-methyl-2-pyrrolidinyl)pyridine (RJ Reynold), MCC or 2-(dimethylamino)ethyl methylcarbamate (Lundbeck), ethyl arecolone or 1-(1,2,5,6-tetrahydro-1-methyl-3-pyridinyl)-1-propanone (Lilly), or isoarecolone or 1-(1,2,3,6-tetrahydro-1-methyl-4-pyridinyl)ethanone (Lilly), or salts, free bases, racemates or enantiomers thereof. Preferably the agonist used is nicotine and most preferably (-)-nicotine.

The term "treatment" used herein relates to both treatment in order to cure or alleviate a disease or a condition, and to treatment in order to prevent the development of a disease or a condition in an acute or in a chronic way.

The term "patient", as it is used herein, relates to any human or non-human mammal in need of the use of a nicotine receptor agonist according to the invention.

The agonist or pharmaceutical preparation according to the invention is to be administered transdermally, sublingually, rectally, intranasally, orally, subcutaneously and/or intramuscularly in any suitable form.

Examples of suitable ways to administer the agonist or pharmaceutical preparation according to the invention is to use a pharmaceutical unit dosage form formulated as a chewing gum, such as Nicorette® gum or a Nicotinell® gum, or as a transdermal patch such as a Nicotinell® patch, or intranasally such as Nicorette® nasal spray. It is also possible to use oral administration and thus a pharmaceutical preparation in the form of e.g. a tablet or a capsule. It may be preferable to use a depot preparation.

The pharmaceutical preparation according to the invention may also comprise other substances, such as an inert vehicle, or pharmaceutical acceptable adjuvants, carriers, preservatives, which are well known to persons skilled in the art.

When the agonist used is nicotine or a pharmaceutical salt thereof, it is preferably administered in a dose from 0.2 mg per day to 60 mg per day for an adult human patient, i.e. from about 0.0028 mg/kg/day to about 0.57 mg/kg/day.

When a Nicorette® or a Nicotinell® chewing gum is used to administer the agonist according to the invention, it may e.g. be used in a dose from 1/4 of a 2 mg chewing gum to 30 2 mg or 15 4 mg gums per day, corresponding to from about 0.0071 mg/kg/day to about 0.57 mg/kg/day.

### Example

The invention is illustrated in this example as the use of nicotine for the manufacture of a medicament for the treatment of a female patient (AA), aged 37, fulfilling DSM-III-R criteria for OCD. AA has had OCD for 13 years. Following the use of nicotine, the patient showed a marked improvement. When evaluating the present example, it should be recalled that the placebo response in OCD is very low (about 10 %), in contrast to other psychiatric disorders, where the placebo response is often up to 50 %. The example is only given in order to illustrate the invention.

### Pre-nicotine status

The intensity of the symptoms in this patient is highest in the morning (before noon) and late at night (1-2 hours before going to bed); several hours during the day can be relatively free of symptoms. The symptoms consist mainly of intrusive thoughts and checking; e g doors, stove and anything that involves cooking and ingestion of food are checked. Typically, practical work is performed with abnormal slowness, whereas intellectual tasks are carried out with speed. AA finds it difficult to initiate and terminate activities.

At best AA is completely free from symptoms several hours during the day, when absorbed by interesting work. At worst, in exhausted or stressful states, the intrusive thoughts are overwhelming enough to impede any form of activity. In this situation AA complains about a high level of "noise" in the form of reverberations of voices and sentences heard previously during the day or in the recent past. There may also be a cavalcade of faces passing by. The intrusive thoughts may also consist of recently performed actions or of actions that are to be carried out shortly. Thoughts/images of these past or future actions blend with thoughts of ongoing actions and disrupt concentration on the ongoing task. Sometimes, thoughts with a diffusely menacing or appalling content appear and disturb focus on an ongoing action. Such thoughts cause considerable distress and anxiety and a sense that everything is whirling around with frantic speed, making focus on any kind of activity impossible. In this situation it is difficult to differentiate between thought and reality; in fact the internal image may be much stronger and more "real" than external images.

The score (comprising items 1-5, obsessions, and items 6-10, compulsions) on the Yale-Brown Obsessive Compulsive Scale (Goodman et al, 1989) before the use of nicotine was 11 on both the obsessions and the compulsions scales, i.e. a total score of 22.

### Post-nicotine status

Initially AA received the 2 mg chewing gum twice a day, one at about 9.30 am and the other at about 3.30 pm. A few weeks later in the course of treatment AA sometimes added a third 2 mg gum at about 7 pm. She did not receive any other pharmacological treatment since a year back, but had previously received SSRI treatment. During the months to follow, she increased the dose gradually to 8-10 nicotine chewing gums per day.

### Beneficial effects

### 8 weeks follow-up

Almost immediately after starting chewing a nicotine gum AA feels more energetic and the threshold for initiating an activity is much lower; it is also much easier to terminate an activity. There are occasions when a task or activity at hand feels impossible to carry through due to fatigue and internal "fog", but shortly after starting chewing the gum, the "fog" and internal chaos dissipate, and the duty can be performed easily and smoothly.

AA also feels much more focused and goal-directed and less distracted by irrelevant stimuli. There is less "noise" in the head and she feels relief as when a fan is turned off. Actions performed appear more distinct. This is a common complaint among OCD patients that the mental registration of performed actions is not distinct enough as to yield a feeling of satisfaction about the performed act; hence the origin of doubt which is a core element in OCD.

There is also less ambivalence and subvalidity. For instance, when she goes shopping, she finds it easier to choose and pick the items she wants to buy; this is true even in stressful situations, i.e. when the store is very crowded.

Another observation AA has made pertains to visual perception: While chewing the nicotine gum she has noticed that it is easier to get an overview of the surroundings. She feels like she "scans" large areas swiftly and smoothly, still perceiving all essential details. Before taking the gum it was as if she had to subdivide the surrounding environment in small sections, and "scan" one at a time, indeed a time-consuming task. In analogy, in the performance of an activity, different components overlap smoothly with one another while taking nicotine, whereas in the pre-nicotine state, an activity was more subdivided into different "segments" (cf. Parkinson's disease).

When chewing the nicotine gum, she also experiences a generally lowered stress level, which she feels is an important feature since she has noticed that stress creates a vicious circle with clouded thinking and loss of concentration leading to more checking, in turn leading to more stress and so forth.

She also feels she has more patience and that she acts friendlier towards others. In addition, there is a general mood elevation and increased ability for hedonia.

The use of nicotine also counteracts episodically occurring uneasiness involving a sense of vague, unidentified threats.

Further, AA has felt more relaxed at night, even if several hours have elapsed since taking the last gum. It is easier to perform various tasks calmly even in states of fatigue, and more time is spent on social activities. Sometimes, though, the patient has taken a third gum at night to further facilitate the performance of various tasks.

Since starting with the use of nicotine AA generally falls asleep earlier. Instead of feeling unease and anxiety at night, she feels sleepy in a relaxed and pleasant way and is less disturbed by various stimuli; for instance she may now fall asleep with the television set on. Moreover she does not feel obligated to check the outer door before going to bed.

The beneficial effects seem to "spill over" to the next day. Even before taking the first gum, the patient feels better and the morning tasks are carried out more swiftly (perhaps partly due to an increased sleep quality; see Salin-Pascual and Drucker-Colin, 1998). If the patient forgets to take the second gum, which has happened on a few occasions, the OCD symptoms are somewhat aggravated again in the evening as well as the following morning.

On a few occasions when the patient has been sleep-deprived and listless and and with unusually many household tasks to tend to, she has consumed 4 gums during one day. This increase in dose made it possible for her to accomplish the duties at hand relatively swiftly and with much less discomfort than usually. AA is very happy about this possibility to be able to adjust the nicotine dose according to current shape and outer demands. With time she has learned to take a gum shortly before a "threshold" is to be overcome; this threshold may consist of getting ready to leave the home or dealing with household matters.

During these first 8 weeks of the use of nicotine when the dosage has been 2-3 2 mg gums/day, there has, apart from the acute and subacute beneficial effects, been a gradual long-term increase in life quality. AA reports that, as the weeks have passed, she feels as if the balance between calm and common-sense thinking (i.e. cognitive powers) and irrational/fearful thinking is continuously and increasingly shifting in favour of the former.

Score (comprising items 1-5, obsessions, and items 6-10, compulsions) on the Yale-Brown Obsessive Compulsive Scale after 8 weeks of the use of nicotine is reduced to 6 on both the obsessions and the compulsions scales, i.e. a total score of 12.

### One year follow-up

One year after starting the use of nicotine, the patient is still very satisfied with the therapy. Her daily nicotine dose has been increased to 8-10 mg/day. The reason for this dose augmentation is that it produces a further increase in stress tolerance, working capacity and functioning in general.

### Adverse effects

The adverse effects generally appear shortly after starting chewing the gum and usually do not last more than 60 minutes.

AA has experienced decreased appetite, sometimes resulting in skipping meals, which may counteract the beneficial effects of nicotine.

She initially experienced slight but tolerable nausea. On one occasion early in the course of using nicotine AA took two gums at a time which resulted in severe nausea and a generally decreased sense of well-being.

During the first weeks of using nicotine she sometimes experienced vertigo/dizziness, especially when quickly moving the head.

Tachycardia and palpitations can be disturbing if the patient is lying down. There may be shortness of breath during exercise.

### Comments on the risk for cardiovascular toxicity

There are several papers discussing the risks for cardiovascular toxicity with the nicotine gum/patch. The plasma concentrations obtained with the gum/patch are generally lower than those obtained by smoking, resulting in weaker cardiovascular effects e g with respect to coronary vascular resistance. According to the literature, the plasma concentration of nicotine achieved 30 minutes after starting chewing the 2 mg gum is about 5 ng/ml (Nicorette, The Nicotine Gum Monograph, Adis Internationsal Limited and Kabi Pharmacia AB, 1992, p 9; cf. Davoli et al, 1998). Cigarette smoking increases blood coagulability, but the nicotine gum/patch does not appear to do so. Secondly, the delivery of carbon monoxide limiting oxygen supply to the heart, is a problem with smoking but not with the nicotine gum/patch (Benowitz and Gourlay, 1997). A recent paper presenting a meta-analysis of 5501 patients receiving the nicotine patch reported no excess of myocardial infarction, stroke, tachycardia, arrhythmia or angina (Greenland et al, 1998). See also Christen and McDonald, 1988.

Hence, the risk for cardiovascular toxicity with the nicotine gum/patch is probably low.

### References

Abood LG, Reynolds DT, Booth H, Bidlack JM (1981) Sites and mechanisms for nicotine's action in the brain. Neuroscience & Biobehavioral Reviews 5: 479-486.

Anderson DJ, Arneric SP (1994) Nicotinic receptor binding of [³H] cytisine, [³H]nicotine and [³H]methylcarbamylcholine in rat brain. Europ J Pharmacol 253: 261-267

Benowitz NL, Gourlay SG (1997) Cardiovascular toxicity of nicotine: Implications for nicotine relacement therapy. J Am Coll Cardiol 29: 1422-1431.

Benowitz NL, Kuyt F, Jacob P III, Jones RT, Osman A-L (1983) Cotinine disposition and effects. Clin Pharmacol Ther 34: 604-611.

Christen AG, McDonald JL Jr (1988) Safety of nicotine-containing gum. Prog Clin Biol Res 261: 219-235.

Crooks PA, Dwoskin LP (1997) Contribution of CNS nicotine metabolites to the neuropharmacological effects of nicotine and tobacco smoking. Biochem Pharmacol 54: 743-753.

Davoli E, Stramare L, Fanelli R, Diomede L, Salmona M (1998) Rapid solid-phase extraction method for automated gas chromatographic-mass spectrometric determination of nicotine in plasma. J Chromatography B 707: 312-316.

Dursun SM, Reveley MA, Bird R, Stirton F (1994) Longlasting improvement of Tourette's syndrome with transdermal nicotine.The Lancet 344: 1577.

Goldberg SR, Risner ME, Stolerman IP, Reavill C, Garcha HS (1989) Nicotine and some related compounds: effects on schedule-controlled behaviour and discriminative properties in rats. Psychopharmacology 97: 295-302.

Goodman WK, Rasmussen SA, Price LH, Mazure D, Heninger GR, Charney DS (1989) The Yale-Brown Obsessive-Compulsive Scale. Arch Gen Psychiatry 46: 1006-1100.

Greenland S, Satterfield MH, Lanes SF (1998) A meta-analysis to assess the incidence of adverse effects associated with the transdermal nicotine patch. Drug Safety 18: 297-308.

Keenan RM, Hatsukami DK, Pentel PR, Thompson TN, Grillo MA (1994) Clin Pharmacol Ther 55: 581-590.

Lin N-H, Meyer MD (1998) Recent developments in neuronal nicotinic acetylcholine receptor modulators. Exp Opin Ther Patents 8: 991-1015.

McConville BJ, Sanberg PR, Fogelson MH, King J, Cirino P, Parker KW, Norman AB (1992) The effects of nicotine plus haloperidol compared to nicotine only and placebo nicotine only in reducing tic severity and frequency in Tourette's disorder. Biol Psychiatry 31: 832-840.

Mitchell SN, Brazell MP, Joseph MH, Alavijeh MS, Gray JA (1989) Regionally specific effects of acute and chronic nicotine on rates of catecholamine and 5-hydroxytryptamine synthesis in rat brain. Europ J Pharmacol 167: 311-322.

Radek RJ (1993) Effects of nicotine on cortical high voltage spindles in rats. Brain Res 625: 23-28.

Rolf D (1998) Use of cotinine in treating psychiatric disorders. US patent 5,776,956.

Rosecrans JA (1979) Nicotine as a discriminative stimulus to behavior: its characterization and relevance to smoking behavior. In: Cigarette Smoking as a Dependence Process, National Institute on Drug Abuse Research, Monograpgh 23, ed by NA Krasnegor, US Department of Health, Education and Welfare, Rockville, MD, pp 58-69.

Salin-Pascual RJ, Drucker-Colin R (1998) A novel effect of nicotine on mood and sleep in major depression. NeuroReport 9: 57-60.

Silver AA, Sanberg PR (1993) Transdermal nicotine patch and potentiation of haloperidol in Tourette's syndrome. Lancet 342: 182

Toth E, Sershen H, Hashim A, Vizi ES, Lajtha A (1992) Effect of nicotine on extracellular levels of neurotransmitters assessed by microdialysis in various brain regions: role of glutamic acid. Neurochem Res 17: 265-271.

Yoshida K, Imura H (1979) Nicotinic cholinergic receptors in brain synaptosomes. Brain Res 172: 453-459.

## Claims

1. Use of a nicotine receptor agonist selected from nicotine, ethyl nicotine, 3-ethynyl-5-(1-methyl-2-pyrrolidinyl)pyridine, 4-[[2-(1-methyl-2-pyrrolidinyl)ethyl]thio]phenol, (S)- 3-ethynyl-5-(1-methyl-2-pyrrolidinyl)pyridine, (2S-trans)-3-(1,4-dimethyl-2-pyrrolidinyl)pyridine, 2-methyl-3-[(2S)-2-pyrrolidinylmethoxy]pyridine, 3-methyl-5-[(2S)-1-methyl-2-pyrrolidinyl]isoxazole, 3-(1-pyrrolidinylmethyl)-pyridine, (3E)-N-methyl-4-(3-pyridinyl)-3-buten-1-amine, 3,4,5,6-tetrahydro-2,3'-bipyridine, (S)-5-fluoro-3-(1-methyl-2-pyrrolidinyl)pyridine, 2-(dimethylamino)ethyl methylcarbamate, 1-(1,2,5,6-tetrahydro-1-methyl-3-pyridinyl)-1-propanone, and 1-(1,2,3,6-tetrahydro-1-methyl-4-pyridinyl)ethanone, or salts, free bases, racemates or enantiomers thereof, for the production of a pharmaceutical preparation for the treatment of obsessive compulsive disorder.

2. Use according to claim 1, wherein said agonist is nicotine or a pharmaceutical salt thereof.

3. Use according to claim 1 or claim 2, wherein said agonist is (-)-nicotine or a pharmaceutical salt thereof.

4. Use according to any one of the claims 1-3, wherein said pharmaceutical preparation is formulated as a chewing gum.

5. Use according to any one of the claims 1-3, wherein said pharmaceutical preparation is formulated for transdermal administration.

6. Use according to any one of the claims 1-3, wherein said pharmaceutical preparation is formulated for oral administration.

7. Use according to any one of the claims 1-3, wherein said pharmaceutical preparation is formulated for sublingual administration.

8. Use according to any one of the claims 1-3, wherein said pharmaceutical preparation is formulated for rectal administration, intranasal administration, subcutaneous administration or intramuscular administration.

9. Use according to any one of the claims 1-3, wherein said pharmaceutical preparation is formulated as a depot preparation.

10. Use according to any one of the claims 2-9, wherein the nicotine or a pharmaceutical salt thereof is used in a dosage unit from 0.2 mg per day to 60 mg per day, corresponding to from 0.0028 mg/kg/day to 0.57 mg/kg/day.

## Patentansprüche

1. Verwendung eines Nikotinrezeptor-Agonisten ausgewählt aus Nikotin, Ethylnikotin, 3-Ethynyl-5-(1-methyl-2-pyrrolidinyl)pyridin, 4-[[2-(1-Methyl-2-pyrrolidinyl)ethyl]thio]phenol, (S)-3-Ethynyl-5-(1-methyl-2-pyrrolidinyl)pyridin, (2S-trans)-3-(1, 4-Dimethyl-2-pyrrolidinyl)pyridin, 2-Methyl-3-[(2S)-2-pyrrolidinylmethoxy]pyridin, 3-Methyl-5-[(2S)-1-methyl-2-pyrrolidinyl]isoxazol, 3-(1-Pyrrolidinylmethyl)-pyridin, (3E)-N-Methyl-4-(3-pyridinyl)-3-buten-1-amin, 3,4,5,6-Tetrahydro-2,3'-bipyridin, (S)-5-Fluoro-3-(1-methyl-2-pyrrolidinyl)pyridin, 2-(Dimethylamino)ethylmethylcarbamat, 1-(1,2,5,6-Tetrahydro-1-methyl-3-pyridinyl)-1-propanon, und 1-(1,2,3,6-Tetrahydro-1-methyl-4-pyridinyl)ethanon, oder Salzen, freien Basen, Racematen oder Enantiomeren davon, für die Herstellung einer pharmazeutischen Zubereitung für die Behandlung von Zwangsneurosen.

2. Verwendung nach Anspruch 1, wobei der Agonist Nikotin oder ein pharmazeutisches Salz davon ist.

3. Verwendung nach Anspruch 1 oder 2, wobei der Agonist (-)-Nikotin oder ein pharmazeutisches Salz davon ist.

4. Verwendung nach einem der Ansprüche 1 bis 3, wobei die pharmazeutische Zubereitung als ein Kaugummi formuliert wird.

5. Verwendung nach einem der Ansprüche 1 bis 3, wobei die pharmazeutische Zubereitung für die transdermale Verabreichung formuliert wird.

6. Verwendung nach einem der Ansprüche 1 bis 3, wobei die pharmazeutische Zubereitung für die orale Verabreichung formuliert wird.

7. Verwendung nach einem der Ansprüche 1 bis 3, wobei die pharmazeutische Zubereitung für die sublinguale Verabreichung formuliert wird.

8. Verwendung nach einem der Ansprüche 1 bis 3, wobei die pharmazeutische Zubereitung für die rektale Verabreichung, intranasale Verabreichung, subkutane Verabreichung oder intramuskuläre Verabreichung formuliert wird.

9. Verwendung nach einem der Ansprüche 1 bis 3, wobei die pharmazeutische Zubereitung als eine Depotzubereitung formuliert wird.

10. Verwendung nach einem der Ansprüche 2 bis 9, wobei das Nikotin oder ein pharmazeutisches Salz davon, in einer Dosierungseinheit von 0,2 mg pro Tag bis 60 mg pro Tag, entsprechend 0,028 mg/kg/Tag bis 0,57 mg/kg/Tag, verwendet wird.

## Revendications

1. Utilisation d'un agoniste du récepteur de la nicotine choisi parmi la nicotine, l'éthylnicotine, la 3-éthynyl-5-(1-méthyl-2-pyrrolidinyl)pyridine, le 4-[[2-(1-méthyl-2-pyrrolidinyl)éthyl]thio]phénol, la (S)-3-éthynyl-5-(1-méthyl-2-pyrrolidinyl)pyridine, la (2S-trans)-3-(1,4-diméthyl-2-pyrrolidinyl)pyridine, la 2-méthyl-3-[(2S)-2-pyrrolidinylméthoxy]pyridine, le 3-méthyl-5-[(2S)-1-méthyl-2-pyrrolidinyl]isoxazole, la 3-(1-pyrrolidinylméthyl)pyridine, la (3E)-N-méthyl-4-(3-pyridinyl)-3-butèn-1-amine, la 3,4,5,6-tétrahydro-2,3'-bipyridine, la (S)-5-fluoro-3-(1-méthyl-2-pyrrolidinyl)pyridine, le méthylcarbamate de 2-diméthylaminoéthyle, la 1-(1,2,5,6-tétrahydro-1-méthyl-3-pyridinyl)-1-propanone et la 1-(1,2,3,6-tétraydro-1-méthyl-4-pyridinyl)éthanone, ou leurs sels, bases libres, racémates ou énantiomères, pour la production d'une préparation pharmaceutique pour le traitement des troubles obsessionnels compulsifs.

2. Utilisation selon la revendication 1, dans laquelle ledit agoniste est la nicotine ou un de ses sels pharmaceutiques.

3. Utilisation selon la revendication 1 ou la revendication 2, dans laquelle ledit agoniste est la (-)-nicotine ou un de ses sels pharmaceutiques.

4. Utilisation selon l'une quelconque des revendications 1 à 3, dans laquelle ladite préparation pharmaceutique est formulée sous la forme d'un chewing-gum.

5. Utilisation selon l'une quelconque des revendications 1 à 3, dans laquelle ladite préparation pharmaceutique est formulée sous la forme d'une administration transdermique.

6. Utilisation selon l'une quelconque des revendications 1 à 3, dans laquelle ladite préparation pharmaceutique est formulée pour une administration orale.

7. Utilisation selon l'une quelconque des revendications 1 à 3, dans laquelle ladite préparation pharmaceutique est formulée pour une administration sub-linguale.

8. Utilisation selon l'une quelconque des revendications 1 à 3, dans laquelle ladite préparation pharmaceutique est formulée pour une administration rectale, une administration intranasale, une administration sous-cutanée ou une administration intramusculaire.

9. Utilisation selon l'une quelconque des revendications 1 à 3, dans laquelle ladite préparation pharmaceutique est formulée sous la forme d'une préparation retard.

10. Utilisation selon l'une quelconque des revendications 2 à 9, dans laquelle la nicotine ou son sel pharmaceutique est utilisé à une dose unitaire de 0,2 mg par jour à 60 mg par jour, correspondant à 0,0028 mg/kg/jour à 0,57 mg/kg/jour.
